# FASCICULE DE BREVET EUROPEEN

(11) **EP 0 678 499 B1**
(45) Date de publication et mention de la délivrance du brevet: **29.10.1997**
(21) Numéro de dépôt: 95400728.2
(22) Date de dépôt: 03.04.1995
(51) Int. Cl.: C07C 227/18, C07C 229/24

(54) **Procédé de préparation d'acide L-aspartique à partir d'aspartate d'ammonium**
Verfahren zur Herstellung von L-Asparaginsäure aus Ammoniumaspartat
Process for the preparation of L-aspartic acid from ammonium aspartate

(30) Priorité: 20.04.1994 FR 9404714
(43) Date de publication de la demande: 25.10.1995
(73) Titulaire: RHONE-POULENC CHIMIE, 92408 Courbevoie Cédex (FR)
(72) Inventeur: Pilot, André, F-79370 Celles Sur Belle (FR); Nore, Olivier, F-79370 Beaussais (FR)
(74) Mandataire: Fabre, Madeleine-France

(56) Documents cités:
- EP-A- 0 588 674
- CRC Handbook of Chemistry and Physics, 69th ed., 1988-89, CRC PRESS, INC., Boca Raion, Florida, US, C-89, C-280

## Description

La présente invention a pour objet un procédé de préparation d'acide L-aspartique par traitement de l'aspartate d'ammonium à l'aide d'une solution alcoolique d'acide fumarique.

Il a été proposé dans la demande de brevet européen EP-A- 588 674, de préparer l'acide L-aspartique par traitement de l'aspartate d'ammonium en milieu aqueux à l'aide d'acide fumarique. Ce traitement au cours duquel l'acide L-aspartique cristallise, peut durer d'environ 10 minutes à plus d'une heure selon les conditions réactionnelles mises en oeuvre. L'acide L-aspartique cristallisé est ensuite séparé par filtration, puis lavé et séché. Le rendement en acide L-aspartique (par rapport à l'aspartate d'ammonium engagé) est de l'ordre de 80% molaire.

La demanderesse a maintenant trouvé que l'emploi d'acide fumarique en solution alcoolique permet d'augmenter le rendement en acide L-aspartique et en outre ne necessite pas d'opération de cristallisation préalable de l'acide L-aspartique avant filtration.

Selon l'invention, il s'agit d'un procédé de préparation d'acide L-aspartique par traitement de l'aspartate d'ammonium à l'aide d'une solution alcoolique d'acide fumarique, selon un rapport molaire acide fumarique ajouté / aspartate d'ammonium présent de l'ordre de 0,05 à 0,8 , de préférence de l'ordre de 0,1 à 0,65 , ladite solution alcoolique renfermant de l'ordre de 1 à 15%, de préférence de 3 à 10%, de son poids d'acide fumarique.

Pour une bonne réalisation du procédé de l'invention, l'aspartate d'ammonium est présent sous forme d'une solution aqueuse de concentration de l'ordre de 0,5 à 2,5 mol/litre, de préférence de l'ordre de 0,8 à 1,8 mol/litre.

Les alcools pouvant être mis en oeuvre pour la mise en solution de l'acide fumarique, sont ceux solvants ou partiellement solvants de l'acide fumarique mais non solvants de l'acide L aspartique.
"Solvants ou partiellement solvants" de l'acide fumarique signifie susceptibles de dissoudre dans les conditions réactionnelles au moins 5% en poids d'acide fumarique. "Non solvants" de l'acide L aspartique signifie susceptibles de dissoudre dans les conditions réactionnelles moins de 0,5% en poids d'acide L aspartique.
Parmi les alcools préférentiels on peut citer le méthanol et l'éthanol.

Le traitement de l'aspartate d'ammonium à l'aide de la solution alcoolique d'acide fumarique peut être réalisé à une température de l'ordre de 20 à 80°C une température de l'ordre de 30 à 55°C convient généralement.

Contrairement au procédé antérieur, l'acide L-aspartique formé précipite et cristallise instantanément. Celui-ci peut être séparé par filtration, puis lavé à l'eau.

Les eaux mères contenant les sels d'ammonium, les acides et l'alcool résiduels sont traités par distillation, afin de récupérer l'acide fumarique et l'alcool.

Le procédé faisant l'objet de l'invention, est particulièrement bien adapté à la préparation d'acide L-aspartique à partir d'aspartate d'ammonium obtenu par traitement enzymatique du fumarate d'ammonium par des aspartases ou des microorganismes producteurs d'aspartases,.tels que *Pseudomonas fluorescens, Escherichia coli* , *Aerobacter aerogenes*, *Bacterium succinium*, *Micrococcus sp*, *Bacillus subtilis et Serratia marcescens*. L'aspartate d'ammonium obtenu à l'issu du traitement enzymatique, est de préférence isolé à la fin de la réaction enzymatique, en vue de l'étape de traitement ultérieure à l'aide de la solution alcoolique d'acide fumarique.

Les exemples suivants sont donnés à titre indicatif.

### Exemple 1

### Préparation du mélange acide fumarique + méthanol

Dans un besher de 500 ml, muni d'un système d'agitation magnétique, on introduiit 200g de méthanol à 99,8%. Cet alcool est porté à 45°C à l'aide d'une plaque chauffante incorporée à l'agitateur magnétique. On introduit ensuite 10% en poids d'acide fumarique (soit 20 g), tout en maintenant la température à 45°C. On obtient ainsi 220 g de solution alcoolique d'acide fumarique.

### Précipitatiion de l'acide aspartique

Dans un erlenmeyer de 500 ml, on introduit 300 g d'une solution aqueuse d'aspartate d'ammonium à 1,15 mole/kg, ce qui représente 0,345 mole. Cette solution est portée à 45°C.
On introduit ensuite les 220 g de solution alcoolique d'acide fumarique préparée ci-dessus, ce qui correspond à 0,172 mole d'acide fumarique et à un rapport molaire acide fumarique/aspartate d'ammonium de 0,5.
Le mélange est agité à l'aide d'un agitateur magnétique.
La température du milieu, qui est de 45°C au moment de la mise en mélange, passe en moins de 15 secondes à 50°C, avec apparition d'un précipité.
Après 15 minutes d'agitation, le mélange est refroidi à 30°C, puis filtré sous vide (2700Pa).
Les cristaux séparés par filtration sont lavés à l'aide de 40 g d'eau distillée. Après séparation du filtrat et séchage des cristaux humides, le poids de cristaux secs obtenus est de 35 g, ce qui correspond à un rendement en acide aspartique de 77%.
Par chromatographie liquide à haute pression, on constate que la pureté des cristaux d'acide aspartique obtenus est de 96,1% ; leur teneur en acide fumarique est de 2,25% et celle en acide malique de 0,07%.

### Exemple 2

### Préparation du mélange acide fumarique + éthanol

Dans un besher de 500 ml, muni d'un système d'agitation magnétique, on introduiit 200g d'éthanol à 95%. Cet alcool est porté à 45°C à l'aide d'une plaque chauffante incorporée à l'agitateur magnétique. On introduit ensuite 7,5% en poids d'acide fumarique (soit 15g), tout en maintenant la température à 45°C. On obtient ainsi 215 g de solution alcoolique d'acide fumarique.

### Précipitatiion de l'acide aspartique

Dans un erlenmeyer de 500 ml, on introduit 224g d'une solution aqueuse d'aspartate d'ammonium à 1,15 mole/kg, ce qui représente 0,259 mole. Cette solution est portée à 45°C.
On introduit ensuite les 215 g de solution alcoolique d'acide fumarique préparée ci-dessus, ce qui correspond à 0,129 mole d'acide fumarique et à un rapport molaire acide fumarique/aspartate d'ammonium de 0,5.
Le mélange est agité à l'aide d'un agitateur magnétique.
La température du milieu, qui est de 45°C au moment de la mise en mélange, passe en moins de 15 secondes à 50°C, avec apparition d'un précipité.
Après 5 minutes d'agitation, le mélange est refroidi à 30°C, puis filtré sous vide (2700Pa).
Les cristaux séparés par filtration sont lavés à l'aide de 108 g d'eau distillée. Après séparation et séchage, le poids de cristaux obtenus est de 25,7 g, ce qui correspond à un rendement en acide aspartique de 75%.
Par chromatographie liquide à haute pression, on constate que la pureté des cristaux d'acide aspartique obtenus est de 98,8% ; leur teneur en acide fumarique est de 2,0% et celle en acide malique de 0,07%.

## Revendications

1. Procédé de préparation d'acide L-aspartique caractérisé en un traitement de l'aspartate d'ammonium à l'aide d'une solution alcoolique d'acide fumarique, selon un rapport molaire acide fumarique ajouté / aspartate d'ammonium présent de l'ordre de 0,05 à 0,8 ladite solution alcoolique renfermant de l'ordre de 1 à 15% de son poids d'acide fumarique.

2. Procédé selon la revendication 1) caractérisé en ce que le rapport molaire acide fumarique ajouté / aspartate d'ammonium présent est de l'ordre de 0,1 à 0,65 , ladite solution alcoolique renfermant de l'ordre de 3 à 10% de son poids d'acide fumarique.

3. Procédé selon la revendication 1) ou 2) caractérisé en ce que l'aspartate d'ammonium est présent sous forme d'une solution aqueuse de concentration de l'ordre de 0,5 à 2,5 mol/litre.

4. Procédé selon la revendication 3) caractérisé en ce que l'aspartate d'ammonium est présent sous forme d'une solution aqueuse de concentration de l'ordre de 0,8 à 1,8 mol/litre.

5. Procédé selon l'une quelconque des revendications précédentes caractérisé en ce que l'alcool mis en oeuvre pour la mise en solution de l'acide fumarique est solvant ou partiellement solvant de l'acide fumarique mais non solvant de l'acide L-aspartique.

6. Procédé selon la revendication 5) caractérisé en ce que l'alcool mis en oeuvre pour la mise en solution de l'acide fumarique est le méthanol ou l'éthanol.

7. Procédé selon l'une quelconque des revendications précédentes caractérisé en ce que le traitement de l'aspartate d'ammonium à l'aide de la solution alcoolique d'acide fumarique est réalisé à une température de l'ordre de 20 à 80°C.

8. Procédé selon la revendication 7) caractérisé en ce que le traitement de l'aspartate d'ammonium à l'aide de la solution alcoolique d'acide fumarique est réalisé à une température de l'ordre de 30 à 55°C.

## Claims

1. Process for the preparation of L-aspartic acid, characterized by a treatment of ammonium aspartate using an alcoholic fumaric acid solution according to an added fumaric acid/ammonium aspartate present molar ratio of the order of 0.05 to 0.8, the said alcoholic solution containing of the order of 1 to 15% of its weight of fumaric acid.

2. Process according to claim 1, characterized in that the added fumaric acid/ammonium aspartate present molar ratio is of the order of 0.1 to 0.65, the said alcoholic solution containing of the order of 3 to 10% of its weight of fumaric acid.

3. Process according to claim 1 or 2, characterized in that the ammonium aspartate is present in the form of an aqueous solution with a concentration of the order of 0.5 to 2.5 mol/litre.

4. Process according to claim 3, characterized in that the ammonium aspartate is present in the form of an aqueous solution with a concentration of the order of 0.8 to 1.8 mol/litre.

5. Process according to any one of the preceding claims, characterized in that the alcohol used for dissolving the fumaric acid is a solvent or partial solvent of fumaric acid but is a non-solvent of L-aspartic acid.

6. Process according to claim 5, characterized in that the alcohol used for dissolving the fumaric acid is methanol or ethanol.

7. Process according to any one of the preceding claims, characterized in that the treatment of ammonium aspartate using the alcoholic fumaric acid solution is carried out at a temperature of the order of 20 to 80°C.

8. Process according to claim 7, characterized in that the treatment of ammonium aspartate using the alcoholic fumaric acid solution is carried out at a temperature of the order of 30 to 55°C.

## Patentansprüche

1. Verfahren zur Herstellung von L-Asparaginsäure, dadurch gekennzeichnet, daß bei einer Behandlung von Ammoniumaspartat mit Hilfe einer alkoholischen Lösung von Fumarsäure gemäß einem molaren Verhältnis zugesetzte Fumarsäure/anwesendes Ammoniumaspartat in der Größenordnung von 0,05 bis 0,8 die genannte alkoholische Lösung Fumarsäure in der Größenordnung von 1 Gew.-% bis 15 Gew.-% umfaßt.

2. Verfahren nach Anspruch 1, dadurch gekennzeichnet, daß das molare Verhältnis zugesetzte Fumarsäure/anwesendes Ammoniumaspartat in der Größenordnung von 0,1 bis 0,65 liegt, wobei die genannte alkoholische Lösung Fumarsäure in der Größenordnung von 3 Gew.-% bis 10 Gew.-% umfaßt.

3. Verfahren nach Anspruch 1 oder 2, dadurch gekennzeichnet, daß das Ammoniumaspartat in Form einer wäßrigen Lösung mit einer Konzentration in der Größenordnung von 0,5 Mol/l bis 2,5 Mol/l vorliegt.

4. Verfahren nach Anspruch 3, dadurch gekennzeichnet, daß das Ammoniumaspartat in Form einer wäßrigen Lösung mit einer Konzentration in der Größenordnung von 0,8 Mol/l bis 1,8 Mol/l vorliegt.

5. Verfahren nach irgendeinem der vorstehenden Ansprüche, dadurch gekennzeichnet, daß der für das Auflösen der Fumarsäure eingesetzte Alkohol die Fumarsäure vollständig oder teilweise, die L-Asparaginsäure jedoch nicht löst.

6. Verfahren nach Anspruch 5, dadurch gekennzeichnet, daß der für das Auflösen der Fumarsäure eingesetzte Alkohol Methanol oder Ethanol ist.

7. Verfahren nach irgendeinem der vorstehenden Ansprüche, dadurch gekennzeichnet, daß die Behandlung des Ammoniumaspartates mit Hilfe der alkoholischen Lösung von Fumarsäure bei einer Temperatur in der Größenordnung von 20 °C bis 80 °C realisiert wird.

8. Verfahren nach Anspruch 7, dadurch gekennzeichnet, daß die Behandlung des Ammoniumaspartates mit Hilfe der alkoholischen Lösung von Fumarsäure bei einer Temperatur in der Größenordnung von 30 °C bis 55 °C realisiert wird.
